# EUROPÄISCHE PATENTANMELDUNG

(11) **EP 2 314 260 A1**
(43) Veröffentlichungstag der Anmeldung: **27.04.2011**
(21) Anmeldenummer: 10013612.6
(22) Anmeldetag: 13.10.2010
(51) Int. Cl.: A61F 13/00, A61F 13/02, A61F 13/08, A61F 13/15

(54) **Material für ein Produkt zur medizinischen Kompressionstherapie sowie eine Bandage und ein Kleidungsstück aus diesem Material**

(30) Priorität: 21.10.2009 DE 102009050031
(71) Anmelder: Christiansen, Hermann, 85139 Wettstetten (DE)
(72) Erfinder: Christiansen, Hermann, 85139 Wettstetten (DE)
(74) Vertreter: Hemmer, Arnd

(57) **Zusammenfassung**

Ein Material für ein Produkt zur Kompression von Körperteilen weist einen flächigen Grundkörper auf. Auf zumindest einer Flachseite dieses Grundkörpers ist mindestens eine linienförmige Erhebung ausgebildet, die sich längs in eine Grundrichtung erstreckt. Die zumindest eine Erhebung weist mindestens einen Abschnitt auf, der schräg und/oder quer zu ihrer Grundrichtung ausgerichtet ist.

## Beschreibung

Die Erfindung betrifft ein Material für ein Produkt zur Kompression von Körperteilen sowie einen Verband und Kleidungsstücke aus diesem Material.

Materialien zur Kompression von Körperteilen werden z.B. bei der medizinischen Kompressionstherapie verwendet, um ein gestörtes bzw. geschädigtes Venen- und/oder Lymphsystem zu entlasten. Hierbei wird mittels einer Bandage oder eines hierzu speziell ausgebildeten Kleidungsstückes, wie z. B. eines Kompressionsstrumpfes ein Druck auf das zu behandelnde Körperteil ausgeübt, um die Entstehung von Ödemen zu verhindern oder um die Größe vorhandener Ödeme mittels Drainage zu verringern.

Es ist üblich, das Material der verwendeten Bandagen und Kleidungsstücke an ihrer an dem Körperteil zur Anlage kommenden Seite zur partiellen Druckerhöhung auf das Körperteil mit Erhebungen zu versehen, um die Wirksamkeit dieser Bandagen und Kleidungsstücke zu verbessern. In diesem Zusammenhang sind solche Erhebungen bekannt, die noppenförmig ausgebildet sind, und solche Erhebungen, die die Form von geraden Linien aufweisen.

Vor dem Hintergrund dieses Standes der Technik liegt der Erfindung die Aufgabe zugrunde, ein Material für ein Produkt zur Kompression von Körperteilen zu schaffen, mit dem sich gegenüber den bislang bekannten Materialien dieser Art verbesserte Drainagewirkungen erzielen lassen. Eine weitere Aufgabe der Erfindung besteht in der Schaffung eines Verbandes und der Schaffung von Kleidungsstücken aus diesem Material.

Gelöst wird diese Aufgabe durch ein Material für ein Produkt zur Kompression von Körperteilen oder ein auf die Haut geklebtes Material mit den in Anspruch 1 angegebenen Merkmalen, durch einen Verband mit den in Anspruch 11 angegebenen Merkmalen und durch ein Kleidungsstück mit den in Anspruch 13 angegebenen Merkmalen. Vorteilhafte Weiterbildungen des Materials, des Verbandes sowie des Kleidungsstücks ergeben sich aus den Unteransprüchen, der nachfolgenden Beschreibung sowie der Zeichnung. Hierbei können gemäß der Erfindung die in den Unteransprüchen und der Beschreibung angegebenen Merkmale jeweils für sich aber auch in Kombination die erfindungsgemäßen Lösungen gemäß den Ansprüchen 1, 11 und 13 weiter ausgestalten.

Das erfindungsgemäße Material für ein Produkt zur Kompression von Körperteilen, das insbesondere für die medizinische Kompressionstherapie vorgesehen ist, weist einen flächigen Grundkörper auf. Auf zumindest einer Flachseite dieses Grundkörpers, die typischerweise zur Anlage an einem Körperteil vorgesehen ist, ist mindestens eine linienförmige Erhebung und vorzugsweise eine Vielzahl solcher Erhebungen ausgebildet, die sich längs in eine Grundrichtung erstrecken. Im Sinne der Erfindung ist unter einem Produkt zur Kompression von Körperteilen nicht nur ein Produkt zur medizinischen Kompressionstherapie, sondern auch jedes zur Anlage an Körperteilen kommende Produkt zu verstehen, das so einen gewissen Druck auf diese Körperteile ausübt. Dies können z. B. auch eng anliegende Bekleidungsstücke o. ä. sein, insbesondere auch Sportbekleidung.

Gemäß der Erfindung weist die zumindest eine Erhebung bzw. weisen die Erhebungen jeweils Abschnitte auf, die schräg und/oder quer zu ihrer Grundrichtung ausgerichtet sind. Das heißt, die zumindest eine Erhebung ist nicht linienförmig gerade ausgebildet, stattdessen weist sie trotz einer klar definierten Grundrichtung einen kurvenförmigen Verlauf mit Ausbuchtungen quer zu der Grundrichtung auf. Die Ausbuchtungen, die jeweils in eine gemeinsame Richtung quer zu der Grundrichtung oder in beide entgegengesetzte Richtungen quer zur Grundrichtung ausgerichtet sein können, können sowohl eckig als auch abgerundet ausgebildet sein. Indem die zumindest eine Erhebung kurvenförmig ausgebildet ist, weist sie gegenüber einer geradlinigen Erhebung bezogen auf einen gleichgroßen Grundkörper eine größere Länge auf. Demzufolge ist die einen Druck auf das zu therapierende Körpergewebe ausübende Fläche bei dem erfindungsgemäßen Material gegenüber dem bislang verwendeten Materialien größer, was zu einer erhöhten Drainagefähigkeit führt. Ein weiterer Vorteil des erfindungsgemäßen Materials ist darin zu sehen, dass die von der zumindest einen Erhebung verdrängte Gewebeflüssigkeit nicht wie bei den bislang gebräuchlichen geradlinigen Erhebungen nur in eine Vorzugsrichtung, das heißt quer zur Längsausdehnung, verdrängt wird, sondern aufgrund der schräg bzw. quer zur Grundrichtung der Erhebung ausgerichteten Bereiche in mehrere unterschiedliche Richtungen geleitet wird und damit besser verteilt abgeführt wird.

Wie bereits angemerkt worden ist, ist es denkbar, den Verlauf der zumindest einen Erhebung in Form einer Zickzack-Linie oder anderweitig eckig auszugestalten. Bevorzugt verläuft die zumindest eine Erhebung allerdings in Grundrichtung mäanderförmig. Dementsprechend hat die Kontur der zumindest einen Erhebung in Draufsicht die Form einer Schlangenlinie mit abgerundeten Schleifen, die sich in die beiden entgegengesetzten Richtungen quer zu der Grundrichtung der Erhebung erstrecken. Der Wölbungsgrad dieser Schleifen ist in Anpassung an die jeweilige Drainageaufgabe grundsätzlich frei wählbar.

Zweckmäßigerweise sind auf dem Grundkörper des erfindungsgemäßen Materials mehrere Erhebungen in deren Grundrichtung nebeneinander ausgebildet. Hierbei können sich die Grundrichtungen der einzelnen Erhebungen durchaus unterscheiden, wobei es aber vorteilhaft ist, wenn sich benachbarte Erhebungen nicht schneiden, sodass der Zwischenraum zwischen benachbarten Erhebungen einen ungehinderten Abflussbereich für die abzuführende Gewebeflüssigkeit bildet. Bevorzugt ist eine Ausgestaltung, bei der benachbarte Erhebungen im Wesentlichen parallel zueinander verlaufen, also jeweils eine gemeinsame Grundrichtung aufweisen.

Die Querschnittskontur der Erhebungen ist prinzipiell ebenfalls beliebig. Bevorzugt ist aber eine Ausgestaltung, bei der die Erhebungen eine normal zur Flachseite des Grundkörpers ausgerichtete, vorzugsweise sinusförmige Wölbung bilden. Die Höhe dieser Wölbungen, das heißt der Abstand des Scheitelpunkts der Wölbungen von dem Grundkörper kann ebenso wie die maximale Breite der Wölbungen in Abhängigkeit von der Drainageaufgabe bis zu 30 mm betragen.

Weiter bevorzugt sind benachbarte Erhebungen voneinander beabstandet angeordnet. Dies ist insofern vorteilhaft, als so ein Zwischenraum zwischen den Erhebungen geschaffen wird, in dem die von den Erhebungen verdrängte Gewebeflüssigkeit ohne äußere Druckbeaufschlagung oder bei zumindest deutlich geringerer äußerer Druckbeaufschlagung abfließen kann. Der Abstand zwischen benachbarten Erhebungen kann vorzugsweise bis zu 30 mm betragen, wobei der letztendlich gewählte Abstand zweckmäßigerweise in Abhängigkeit von der zu erfüllenden Drainageaufgabe zu wählen sein wird. Gegebenenfalls können benachbarte Erhebungen auf einem gemeinsamen Grundkörper auch unterschiedliche Abstände voneinander aufweisen.

Darüber hinaus kann es auch von Vorteil sein, wenn benachbarte Erhebungen, wie es eine weitere bevorzugte Ausgestaltung des erfindungsgemäßen Materials vorsieht, im Wesentlichen direkt aneinander angrenzen. Das heißt, die Erhebungen können quasi direkt nebeneinander oder in einem zu vernachlässigenden Abstand zueinander angeordnet sein. Hierdurch lässt sich ein besonders großer Flächendruck auf das zu behandelnde Gewebe erzielen.

Den Grundkörper des erfindungsgemäßen Materials kann vorteilhaft eine Textilie bilden. Demzufolge kann ein Verbund von natur- und/oder Kunststofffasern, beispielsweise in Form eines Tuches, den Grundkörper bilden. Die Verwendung elastischer Kunststofffasern kann dem Grundkörper hierbei die bei Produkten zur medizinischen Kompressionstherapie in der Regel geforderte Elastizität verleihen.

Daneben kann es auch vorteilhaft sein, wenn der Grundkörper von einer Folie oder einem Schaumstoffkörper gebildet wird. So können z. B. vorzugsweise gasundurchlässige Kunststofffolien zumindest einen zur Anlage an einem Körperteil bestimmten Teil eine aufblasbare Kompressionsmanschette bilden. Die Verwendung eines Schaumstoffkörpers als Grundkörper des erfindungsgemäßen Materials hat beispielsweise den Vorteil, dass ein solcher Schaumstoffkörper sehr einfach herstellbar ist und gegebenenfalls auch einfach weiterverarbeitet werden kann.

Die zumindest eine Erhebung kann auf dem Grundkörper vorteilhafterweise aufgetragen sein. So kann die Erhebung auf der makroskopisch gesehen glatten Oberfläche des Grundkörpers zum Beispiel aufgespritzt oder aufgeklebt sein und vorzugsweise bei textilen Grundkörpern auch auf dem Grundkörper aufgestickt sein.

Daneben kann die zumindest eine Erhebung vorteilhaft auch einen Teil des Grundkörpers bilden, also ein integraler Bestandteil des Grundkörpers sein. Eine solche Ausgestaltung bietet sich insbesondere dann an, wenn eine Kunststofffolie oder ein Schaumstoffkörper den Grundkörper bilden, da die Erhebung bzw. die Erhebungen bei solchen Grundkörpern bereits in einfacher Weise bei der Herstellung des Grundkörpers, das heißt, der Kunststofffolie bzw. des Schaumstoffkörpers, an diesem Grundkörper ausgeformt werden können.

Die Erfindung betrifft auch alle Arten von Produkten zur Kompression von Körperteilen aus einem Material gemäß der vorangehenden Beschreibung. So betrifft die Erfindung auch einen Verband. Unter Verbänden sind im Sinne der Erfindung alle Mittel zu verstehen, mit denen ein Körperteil flächig abgedeckt werden kann, um es beispielsweise vor schädlichen Umwelteinflüssen oder mechanischer Belastung zu schützen, oder die zur Kompression, Blutstillung, Sekretaufnahme oder Okklusion dienen. Demzufolge kann es sich vorzugsweise um medizinische Verbände z.B. in Form von Binden, Bandagen und Wundauflagen oder um im Bereich des Sports zur Prävention von Verletzungen verwendete Verbände handeln.

Der erfindungsgemäße Verband, insbesondere medizinische Verband, ist zumindest abschnittsweise und bevorzugt in einem Bereich, der zur direkten Anlage an einem Körperteil vorgesehen ist, aus dem erfindungsgemäßen Material ausgebildet, das mindestens eines der oben beschriebenen Merkmale aufweist. Eine Bandage kann als eine um ein Körperteil zu wickelnde Binde oder als eine schlauchförmige Manschette ausgebildet sein. Die Grundabmessungen, das heißt Länge und Breite einer Bandagenbinde sind beliebig wählbar. Die Abmessungen einer als Manschette ausgebildeten Bandage sind typischerweise an Größe und Form des Körperteils anzupassen, an dem sie angelegt werden soll. Bei dem erfindungsgemäßen Verband erstreckt sich die Grundrichtung der zumindest einen Erhebung bevorzugt quer zur Längsausdehnung des Verbandes, wobei unter der Längsausdehnung einer als schlauchförmige Manschette ausgebildeten Bandage deren Umfangsrichtung zu verstehen ist. Die Ausrichtung der Erhebung bzw. Erhebungen an der Bandage ist also bevorzugt derart, dass sich die Erhebung oder Erhebungen und dazwischen ausgebildete Freiräume, wenn die Bandage zum Beispiel an einem Bein angelegt ist, in Längsrichtung des Beines erstrecken. Dies ist insofern vorteilhaft, als von den Erhebungen in das zwischen den Erhebungen liegendes Gewebe verdrängte Gewebeflüssigkeit ungehindert und ggf. schwerkraftunterstützt abfließen kann.

Der Verband kann vorteilhaft als ein Klebeverband ausgebildet sein. So kann beispielsweise eine als Binde ausgebildete Bandage als eine Klebebandage ausgebildet sein. Auf zumindest einem Abschnitt einer Flachseite eines Verbandes bzw. einer Bandage kann ein Klebe- bzw. Haftmittel aufgetragen sein. Hierbei ist es bevorzugt vorgesehen, das Klebemittel auf der Flachseite des Verbandes bzw. der Bandage aufzutragen, die mit den erfindungsgemäßen Erhebungen ausgestattet ist. So ist es möglich, das Klebemittel direkt auf den Erhebungen aufzutragen oder die Zwischenräume zwischen benachbarten Erhebungen als Depot für das Klebemittel zu verwenden. Typischerweise ist es auch möglich, sowohl die Erhebungen als auch die Bereiche zwischen den Erhebungen mit einer Klebeschicht zu versehen. Auch kann das Klebemittel in Form der Erhebungen auf den Verband bzw. auf die Bandage aufgebracht sein, so dass das Klebemittel zumindest einen Teil der Erhebung bildet.

Die Erfindung betrifft weiter ein Kleidungsstück, das zumindest abschnittsweise aus einem Material mit mindestens einer der oben beschriebenen Eigenschaften ausgebildet ist. Ein solches Kleidungsstück kann zumindest in einzelnen Bereichen ein Produkt zur Kompression von Körperteilen bilden. Vorteilhaft kann das Kleidungsstück ein Strumpf sein. Solche Strümpfe sind als Kompressions- oder Stützstrümpfe bekannt und dienen beispielsweise dazu, einen Entstauungszustand in einem Bein zu erhalten. Weiter vorteilhaft kann das erfindungsgemäße Kleidungsstück auch ein Büstenhalter sein, der beispielsweise nach einer brusterhaltenden Brustkrebsoperation eingesetzt werden kann. Nach einer solchen Operation kann es zu Komplikationen wie zum Beispiel zu einem lymphatischen Ödem oder einer Fibrose kommen. Hier kann eine manuelle Lymphdrainage zu einer Linderung führen, wobei zur Aufrechterhaltung dieses Zustands der erfindungsgemäße Büstenhalter eingesetzt werden kann, der zumindest in dem die betroffene Brust aufnehmendem Körbchen mit dem erfindungsgemäßen Material ausgestattet ist.

Darüber hinaus können auch beliebig andere Kleidungsstücke aus dem erfindungsgemäßen Material hergestellt sein. So ist es beispielsweise denkbar, dieses Material auch bei Strumpfhosen, Miedern, Schwimmbekleidung, Unterwäsche, Sportbekleidung, Schlankheitsstützwäsche und dergleichen zu verwenden, da das Material neben den hervorstechenden Drainageeigenschaften beispielsweise auch eine bessere Schweißabsorption, eine Verbesserung der Blutversorgung der Muskulatur und damit eine Verbesserung der muskulären Leistungsfähigkeit bewirken kann. Des Weiteren kann die Oberflächenstruktur des Materials und dessen Stützwirkung auch als ästhetisch ansprechend empfunden werden.

Nachfolgend wird die Erfindung anhand von in den Zeichnungen dargestellten Ausführungsbeispielen näher erläutert. In den Zeichnungen zeigt:
- Fig. 1: in perspektivischer Seitenansicht schematisch ein Material in einem ersten Ausführungsbeispiel,
- Fig. 2: in perspektivischer Seitenansicht schematisch ein Material in einem zweiten Ausführungsbeispiel,
- Fig. 3: in perspektivischer Seitenansicht schematisch ein Material in einem dritten Ausführungsbeispiel,
- Fig. 4: in perspektivischer Seitenansicht schematisch ein Material in einem vierten Ausführungsbeispiel,
- Fig. 5: in perspektivischer Seitenansicht schematisch ein Material in einem fünften Ausführungsbeispiel,
- Fig. 6: in perspektivischer Seitenansicht schematisch ein Material in einem sechsten Ausführungsbeispiel,
- Fig. 7: in perspektivischer Seitenansicht schematisch ein Material in einem siebten Ausführungsbeispiel,
- Fig. 8: in perspektivischer Seitenansicht schematisch ein Material in einem achten Ausführungsbeispiel,
- Fig. 9: in perspektivischer Seitenansicht schematisch ein Material in einem neunten Ausführungsbeispiel,
- Fig. 10: in Draufsicht ein Material in einem zehnten Ausführungs- beispiel,
- Fig. 11: in Draufsicht ein Material in einem elften Ausführungsbei- spiel, und
- Fig. 12: in Draufsicht ein Material in einem zwölften Ausführungs- beispiel.

Die in den Zeichnungen dargestellten Materialien weisen jeweils einen flachen Grundkörper 2 auf, bei dem auf einer Flachseite mehrere Erhebungen 4, 4', 4", 4"', 4^{IV} und 4^{V} aufgebracht sind. Der Grundkörper 2 kann wahlweise von einer Textilie, einer Kunststofffolie oder von einem Schaumstoffkörper gebildet sein. Auch die Erhebungen 4, 4', 4", 4"', 4^{IV} und 4^{V} können eine textile Ausbildung aufweisen, beispielsweise auf dem Grundkörper 2 aufgestickt sein oder aus Kunststoff bestehen und auf dem Grundkörper aufgespritzt oder aufgeklebt sein.

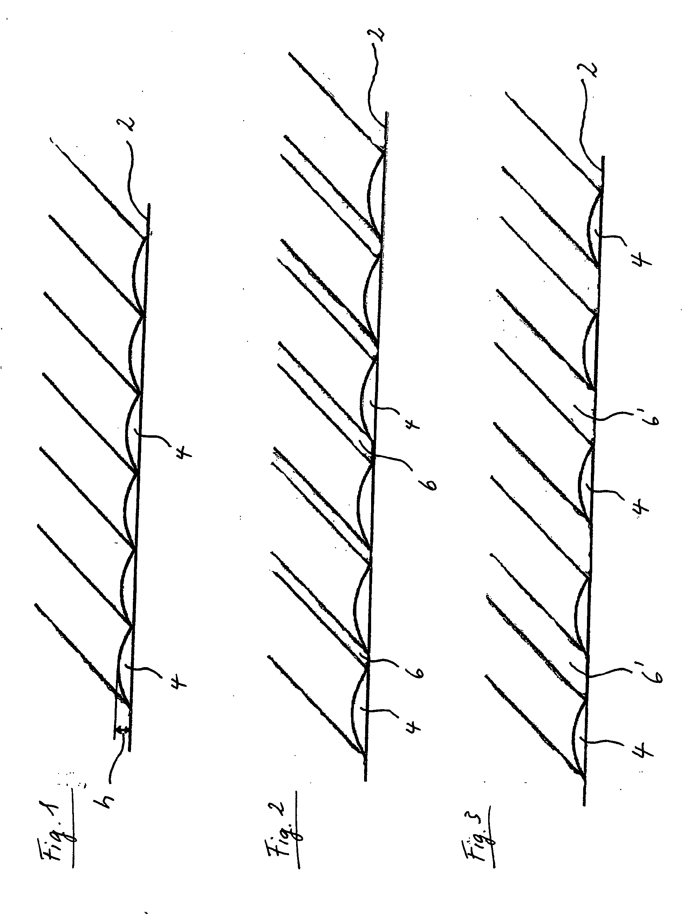

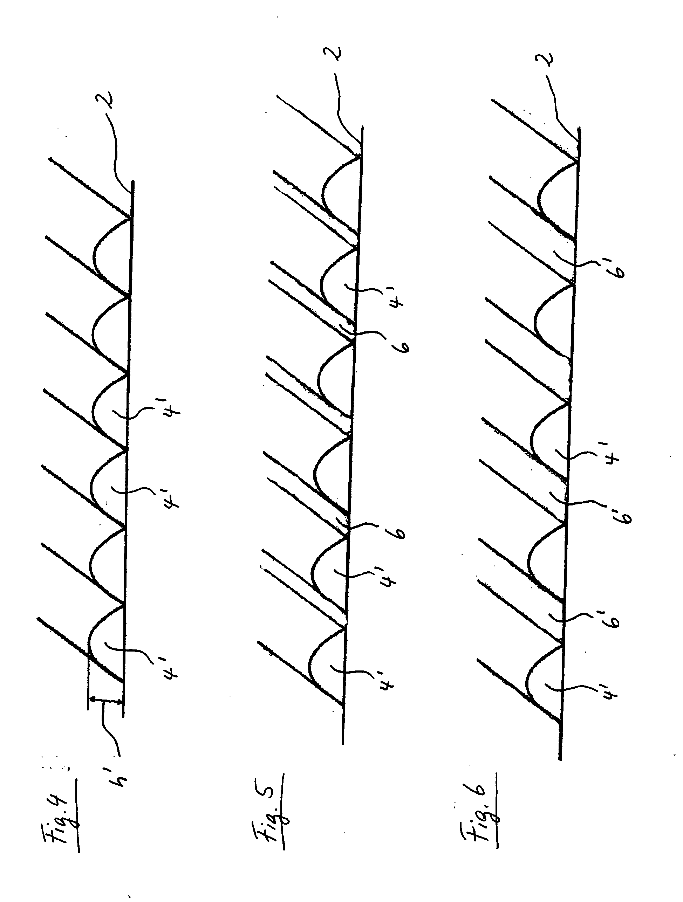

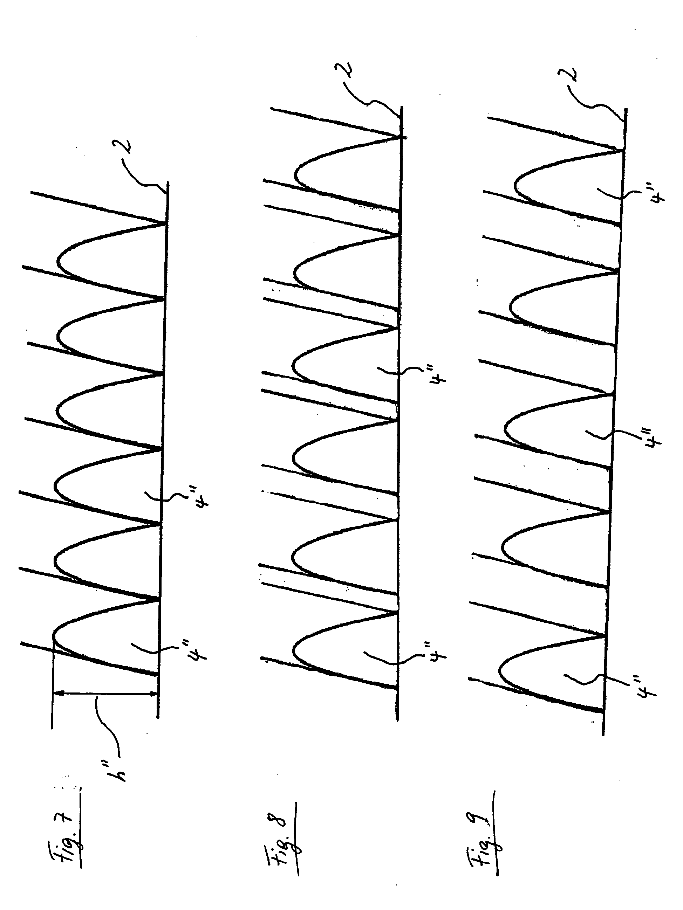

Die in den Figuren 1 bis 3 dargestellten Erhebungen 4, die in den Figuren 4 bis 6 dargestellten Erhebungen 4' und die in den Figuren 7 bis 9 dargestellten Erhebungen 4" sind jeweils sinusförmig gewölbt ausgebildet und unterscheiden sich lediglich hinsichtlich ihrer Höhe h, h' bzw. h". So ist die Höhe h" der Erhebung 4" größer als die Höhe h' der Erhebung 4', die wiederum größer als die Höhe h der Erhebung 4 ist. Die Höhe h, h' bzw. h" ist frei wählbar und kann bis zu 30 mm betragen.

Die in den Figuren 1 bis 3 dargestellten Materialien unterscheiden sich lediglich dahingehend, dass benachbarte Erhebungen 4 in den einzelnen Figuren unterschiedlich beabstandet sind. So sind die Erhebungen 4 bei dem in Figur 1 dargestellten Ausführungsbeispiel direkt aneinander angrenzend angeordnet, während die Erhebungen 4 in den in den Figuren 2 und 3 dargestellten Ausführungsbeispielen voneinander beabstandet sind. So ist zwischen benachbarten Erhebungen 4 bei den Materialien nach den Figuren 2 und 3 ein Zwischenraum 6 bzw. 6' ausgebildet, der bei Einsatz des Materials zum Beispiel in Verbindung mit einer Bandage oder einem therapeutischen Kleidungsstück einen Kanal zum Abführen von Gewebeflüssigkeit bildet. Die Breite des Zwischenraumes 6' in Fig. 3 ist größer als die des Zwischenraums 6 in Fig. 2 und bis zu einer Breite von ungefähr 30 mm grundsätzlich frei wählbar.

Analog unterscheiden sich die in den Fig. 4 bis 6 und die in den Fig. 7 bis 9 dargestellten Materialien durch den Abstand benachbarter Erhebungen 4' bzw. 4". So sind die Erhebungen 4' in Fig. 4 und die Erhebungen 4" in Fig. 7 direkt aneinander angrenzend angeordnet, während zwischen den Erhebungen 4' in Fig. 5 und den Erhebungen 4" in Fig. 8 ein Zwischenraum 6 und zwischen den Erhebungen 4' in Fig. 6 und den Erhebungen 4" in Fig. 9 ein Zwischenraum 6' ausgebildet ist.

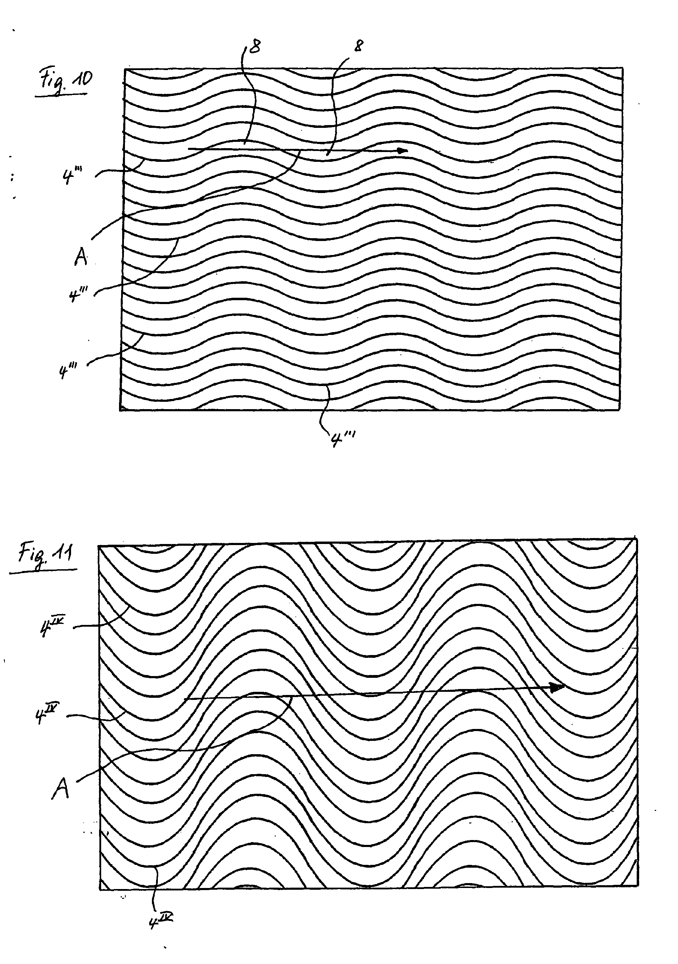

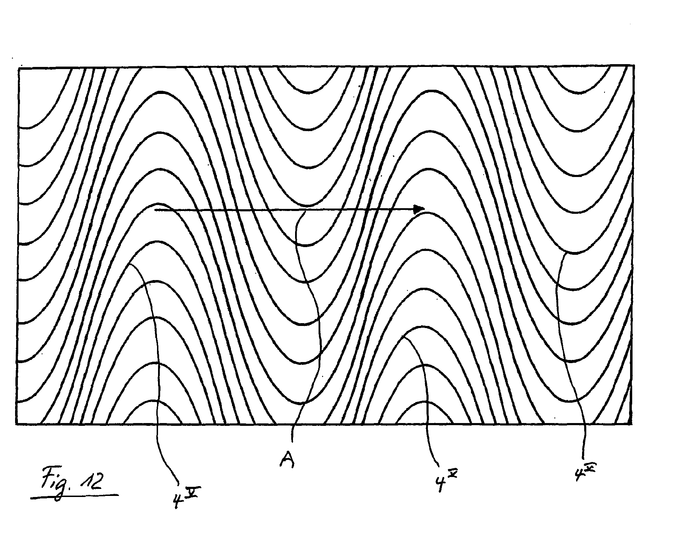

Aus den Fig. 10 bis 12 ist ersichtlich, dass die dort vorgesehenen Erhebungen 4"', 4^{IV} und 4^{V} zwar jeweils in eine Grundrichtung A ausgerichtet sind, jedoch nicht geradlinig in dieser Grundrichtung A verlaufen. Stattdessen haben die Erhebungen 4"', 4^{IV} und 4^{V} in Grundrichtung A einen mäanderförmigen, das heißt schlangenlinienförmigen Verlauf, sodass sie größtenteils schräg zur Grundrichtung A ausgerichtet sind. Bezogen auf die Grundrichtung A der einzelnen Erhebungen 4"', 4^{IV} und 4^{V} bilden die Erhebungen 4"', 4^{IV} und 4^{V} abgerundete Wölbungen 8 (siehe Fig. 10), die sich in die beiden entgegengesetzten Richtungen quer zur Grundrichtung A der Erhebungen 4"', 4^{IV} und 4^{V} erstrecken. Der Wölbungsgrad dieser Wölbungen 8 ist in den Fig. 10 bis 12 Ausführungsbeispielen unterschiedlich und in Anpassung an die jeweilige Drainageaufgabe frei wählbar.

### Bezugszeichenliste

- 2: - Grundkörper
- 4, 4', 4", 4"', 4^{IV}, 4^{V}: - Erhebung
- 6, 6': - Zwischenraum
- 8: - Wölbung
- A: - Grundrichtung
- h, h', h": - Höhe

## Patentansprüche

1. Material für ein Produkt zur Kompression von Körperteilen mit einem flächigen Grundkörper (2), bei dem auf zumindest einer Flachseite mindestens eine linienförmige Erhebung (4, 4', 4", 4"', 4^{IV}, 4^{V}) ausgebildet ist, die sich längs in eine Grundrichtung (A) erstreckt, wobei die Erhebung (4, 4', 4", 4"', 4^{IV}, 4^{V}) Abschnitte aufweist, die schräg und/oder quer zu ihrer Grundrichtung (A) ausgerichtet sind.

2. Material nach Anspruch 1, bei dem die zumindest eine Erhebung (4, 4', 4", 4'", 4^{IV}, 4^{V}) in Grundrichtung (A) mäanderförmig verläuft.

3. Material nach einem der vorangehenden Ansprüche, bei dem auf dem Grundkörper (2) mehrere Erhebungen (4, 4', 4", 4"', 4^{IV}, 4^{V}) in deren Grundrichtung (A) nebeneinander ausgebildet sind.

4. Material nach einem der vorangehenden Ansprüche, bei dem die Erhebungen (4, 4', 4", 4"', 4^{IV}, 4^{V}) eine normal zur Flachseite des Grundkörpers (2) ausgerichtete vorzugsweise sinusförmige Wölbung (8) bilden.

5. Material nach einem der vorangehenden Ansprüche, bei dem benachbarte Erhebungen (4, 4', 4", 4"', 4^{IV}, 4^{V}) voneinander beabstandet sind.

6. Material nach einem der Ansprüche 1 bis 4, bei dem benachbarte Erhebungen (4, 4', 4", 4"', 4^{IV}, 4^{V}) im Wesentlichen direkt aneinander angrenzen.

7. Material nach einem der vorangehenden Ansprüche, bei dem der Grundkörper (2) eine Textilie ist.

8. Material nach einem der vorangehenden Ansprüche, bei dem der Grundkörper (2) eine Folie oder ein Schaumstoffkörper ist.

9. Material nach einem der vorangehenden Ansprüche, bei dem die zumindest eine Erhebung (4, 4', 4", 4"', 4^{IV}, 4^{V}) auf den Grundkörper (2) aufgetragen ist.

10. Material nach einem der Ansprüche 1 bis 8, bei dem die zumindest eine Erhebung (4, 4', 4", 4"', 4^{IV}, 4^{V}) einen Teil des Grundkörpers (2) bildet.

11. Verband, der zumindest abschnittsweise aus einem Material mit mindestens einem in den Ansprüchen 1 bis 10 angegebenen Merkmalen ausgebildet ist.

12. Verband nach Anspruch 11, bei der sich die Grundrichtung der zumindest einen Erhebung quer zur Längsausdehnung der Bandage erstreckt.

13. Kleidungsstück, das die zumindest abschnittsweise aus einem Material mit mindestens einem in den Ansprüchen 1 bis 10 angegebene Merkmalen ausgebildet ist.

14. Kleidungsstück nach Anspruch 12, welches ein Strumpf ist.

15. Kleidungsstück nach Anspruch nach Anspruch 12, welches ein Büstenhalter ist.
